# EUROPEAN PATENT SPECIFICATION

(11) **EP 2 903 540 B1**
(45) Date of publication and mention of the grant of the patent: **25.07.2018**
(21) Application number: 13727410.6
(22) Date of filing: 14.03.2013
(51) Int. Cl.: A61B 17/42

(54) **MEDICAL INSTRUMENT FOR CONTROLLED UTERINE MANIPULATION**
MEDIZINISCHES INSTRUMENT ZUR KONTROLLIERTEN GEBÄRMUTTERMANIPULATION
INSTRUMENT MÉDICAL POUR LA MANIPULATION CONTRÔLÉE DE L'UTÉRUS

(30) Priority: 14.03.2012 SI 201200084
(43) Date of publication of application: 12.08.2015
(73) Proprietor: Tomazevic, Rok, 1000 Ljubljana (SI)
(72) Inventor: TOMAZEVIC, Tomaz Kristijan, 1000 Ljubljana (SI)
(86) International application number: PCT/SI2013/000012
(87) International publication number: WO 2013/151512

(56) References cited:
- EP-A1- 2 116 202
- WO-A1-2008/074054
- WO-A1-2011/140604
- WO-A2-03/015643
- US-A1- 2003 187 334

## Description

The object of the invention is a medical instrument providing a simple and safe uterine manipulation during gynaecologic endoscopic surgical procedures.

The device consists of four parts: a guiding cylindrical rod at one side ending with a snail - coiled cone, a coaxial cylinder with cervical cup, a female cylindrical occluding screw and a pear shaped female occluding screw for lower vaginal occlusion. The invention belongs to the class A61B/17/42 of the International Patent Classification.

The problem, which has successfully been solved by presented construction of the instrument for controlled uterine manipulation, was a problem of constructing such an reusable instrument that is easy to use and fulfils almost all demands required during uterine manipulation, adapts to the individual anatomy of the patient, enables good uterine elevation, appropriate changes of uterine positions, good presentation of operative field, good access with operative instruments, a clear delineation of vaginal fornices, a tight vaginal occlusion, and therefore reduces the problem of inappropriate assistance during gynaecologic endoscopic surgical procedures, increases operative safety and decreases the operative time.

The assistance by means of uterine manipulator plays crucial role during total and subtotal laparoscopic hysterectomy. Appropriate uterine manipulation improves assistance and helps surgeons to expose anatomical and pathological structures of uterus, adnexa, blood vessels, uropoetic organs and intestines, enlarges distances between genital, uropoetic and intestinal organs and during laparoscopic total and subtotal histerectomy delineates vaginal fornices and at the same time, even after surgical opening of the vagina, provides a good pneumo occlusion.

Many existing instruments intend to fulfil numerous demands of an appropriate uterine manipulation, but the ideal uterine manipulator to optimize the assistance during endoscopic gynaecologic operative procedures has not been developed yet. The existing manipulators importantly differ in appearance, materials, weight, complexity of assembling, functioning, in adapting to an individual anatomy of a patient, in pneumo occlusion. No single uterine manipulator seems to have all the attributes of an ideal manipulator. Certain types of uterine manipulators are reusable, some are even disposable.

Such kinds of manipulators are described in various Patent Documents such as US 2009/0137970, US 2010/0305578, US 2012/0109147, US2012/0109146 and US2010/0180422. Document WO03015643 A2 discloses a device for manipulating the uterus according to the preamble of claim 1. The object of the invention relates to a medical instrument for controlled uterine manipulation during gynaecologic endoscopic operative procedures and comprises of a smooth cylindrical guiding part at one side ending with a snail coiled cone, of a coaxially movable cylinder with a cervical cup, of a cylindrical occluding female screw and of a pear shaped occluding female screw for vaginal occlusion . The present invention is defined by appended claim 1. Specific embodiments are set forth in the dependent claims. The object of the invention, a medical instrument for controlled uterine manipulation during gynaecologic endoscopic operative procedures will be explained in detail and presented with a sample and with the following figures:
- **Figure 1**: medical instrument for controlled uterine manipulation according to invention - axonometric view;
- **Figure 2**: guiding part of the instrument - according to invention
- **Figure 3, 4, 5**: coaxially movable cylinder with a cervical cup-according to invention
- **Figure 6,7,8**: pear shaped female screw for occlusion of lower vagina - according to invention
- **Figure 9**: occluding female screw - according to invention

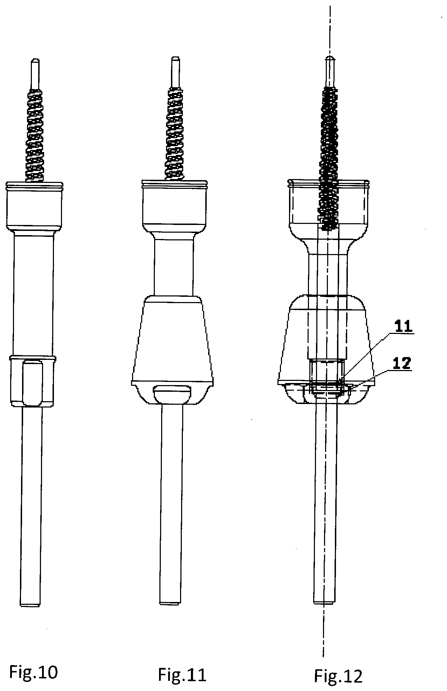

- **Figure 10,11,12**: three different combinations of cylinders with cervical cups of different sizes and of occluding female screw alone or enlarged with a occluding pear shaped female screws for occlusion of lower vagina of different sizes can be adapted to anatomy of a patient.

The medical instrument for controlled uterine manipulation according to the invention is composed of a guiding part - a smooth metallic cylindrical rod 1 at one end undergoing to a snail coiled cone 5 with cylindrical end 4, of a coaxially situated movable cylinder with a cervical cup 2, of a simple cylindrical locking nut 9 which is enlarged with a pear shaped nut 3 for occlusion of lower vagina.

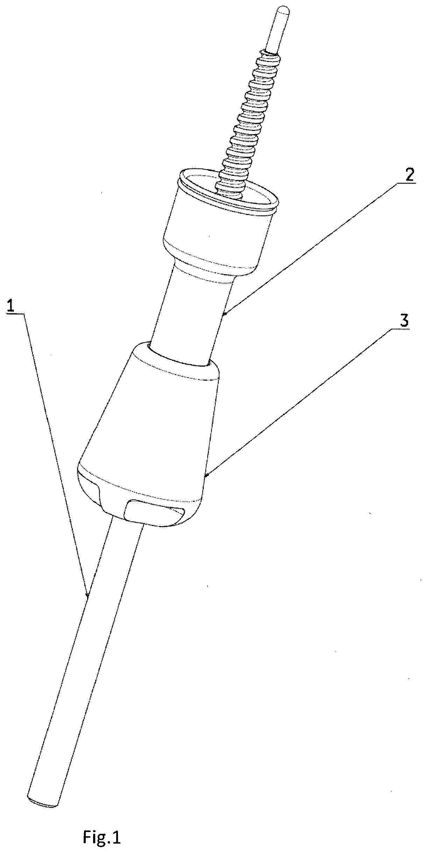

We continue with a presentation and a description of components of the instrument for controlled uterine manipulation according to inventions. Its axonometric view with preliminary dimensions not limiting an invention is presented in the figure 1.

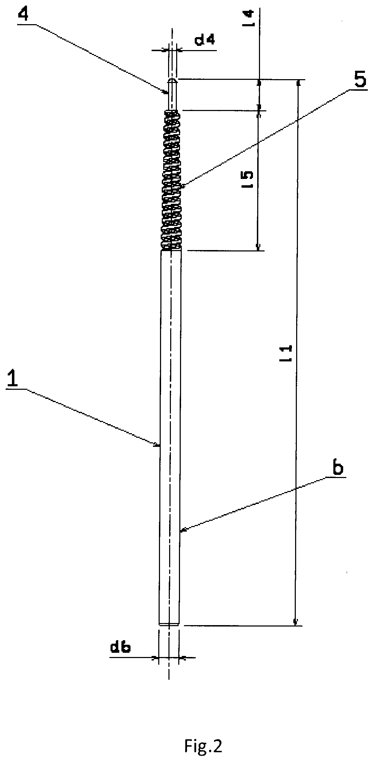

Figure 2 shows a cylindric guiding rod 1, 13 mm in diameter and 352 mm in length. A smooth cylindrical rod 1, 242mm in length, 13 mm in diameter undergoes to a snail coiled conic part 5 90 mm in length, ending with a 20 mm long cylinder 5 mm in diameter with a rounded end 4. The guiding part 1 is preferably made either of stainless medical steel, or of teflon or teflon similar materials.

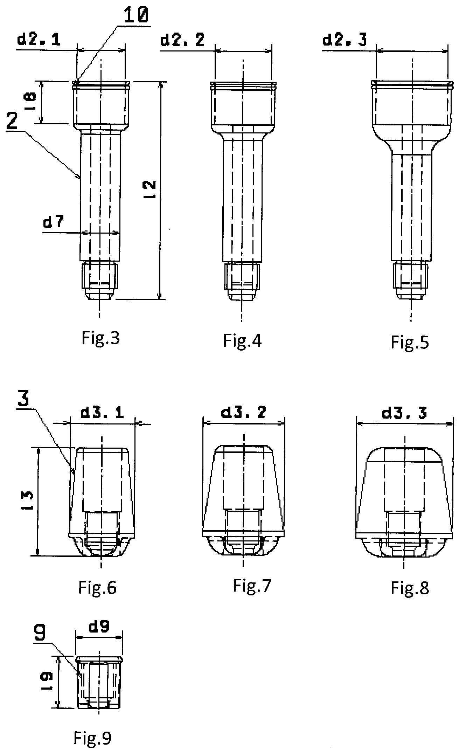

Figures 3, 4 and 5 show three coaxially movable cylinders with a cervical cup 2. Cervical cups with grooved outer margins 10 are made in three diameters (31, 36 and 46 mm). All pieces have the same length (140 mm) and the same diameter 25 mm and can therefore be interchanged. Regardless of their diameter, the depths of three different cups 2 are the same - 28 mm. They are preferably made of teflon or teflon similar material.

Figure 9 shows a cylindrical occluding teflon made female screw. It serves to fix a coaxially movable cylinder with cervical cup to a guiding rod. The point of fixation can be chosen individually - according to individual anatomic position. The small outer nut on a surface of occluding nut 3 serves for fixation of an occluding pear shaped vaginal obturator. It is preferably made of teflon or teflon similar material.

The pear shaped nuts 3 for occlusion of lower vagina obturators are presented in the figures 6,7,8. Outer diameters of different sized obturators are 42 mm, 52 mm and 62 mm. Regardless of their diameter, the lengths of the obturators are the same - 70 mm. Internal nuts of all pear shaped obturators have the same diameter and correspond to the small outer screw on the surface of the female nut. They are interchangeable and can be coaxially fixed to the outer surface of the nut 3. The dimension of a pear shaped obturator can be chosen according to individual anatomy of a patient. It provides a tight pneumo occlusion and provides a good visibility during the phase of operative opening of the vagina. It is preferably made of teflon or teflon similar material.

The application of the instrument starts with the rotatory introduction and fixation of the conical snail screwed part of the guiding rod 1 through the cervical canal to the uterus. Next step is coaxial introduction of the cylinder with the cervical cup 2 high up to vaginal fornices. When it is in a good position, the screwed end of cylindrical part 3, 4, 5 is firmly fixed with a cylindrical occluding locking nut 9 to the guiding rod 2. The small outer diameter of a cylindrical female screw can be enlarged by rotatory introduction and fixation of a pear shaped nut 3 for occlusion of lower vagina. Appropriate selection of composing parts enables individualised adaptation of the instrument to anatomy of a patient.

Appropriate fixation of the cylinder with a cervical cup 2 to the previously fixed guiding rod 1 enables an appropriate uterine manipulation and a good visualisation of pelvic structures. Grooved outer margins of cervical cup delineate vaginal fornices and also provide a good pneumo occlusion. Additionally the use of pear shaped nut 3 for occlusion of lower vagina provides a tight pneumo occlusion and conserves a good visibility of pelvic structures even after opening of the vagina. The medical instrument for controlled uterine manipulation is made of materials which allow multiple sterilisations. Controlled uterine manipulation with a reusable instrument reduces the expenses of the gynaecological endoscopic operations.

## Claims

1. A medical instrument for controlled uterine manipulation comprising
- a guiding rod (1) comprising a proximal cylindrical rod-shaped part, a cylindrical distal end (4) and a threaded conical portion (5) between the proximal cylindrical rod-shaped part (6) and the cylindrical distal end (4),
- a cervical cup (2) coaxially movable along the guiding rod,
- a cylindrical locking nut (9),
wherein the threaded conical portion of the guiding rod (1) is configured to be fixed to the uterus through the cervical canal, wherein the cervical cup (2) comprises a cylinder with a screwed proximal end configured to be fixed to the guiding rod at a point of fixation that can be chosen individually, the cervical cup being further configured to be coaxially introduced over the threaded conical portion high up to the vaginal fornices, **characterised in that** the medical instrument further comprises a pear-shaped nut (3), **in that** the cervical cup (2) further comprises outer grooved margins (10) to delineate vaginal fornices and provide pneumo occlusion, **in that** the cylindrical locking nut (9) is configured to be screwed to the said screwed proximal end of the cylinder of the cervical cup to fix the cylinder to the guiding rod, **in that** the cylindrical locking nut (9) further comprises an outer screw on its surface, for fixation of the pear-shaped nut (3), **in that** the pear-shaped nut(3) is configured for occlusion of the lower vagina and is configured to be fixed coaxially to the outer surface of the cylindrical locking nut.

2. A medical instrument for controlled uterine manipulation according to claim 1,
**characterised in that**, the cylinder with a cervical cup (2), the cylindrical locking nut (9) and the pear-shaped nut (3) are made of PTFE.

3. A medical instrument for controlled uterine manipulation according to claim 1 and 2,
**characterised in that**, the guiding rod (1) is made either of stainless medical steel or PTFE.

## Patentansprüche

1. Ein medizinisches Instrument zur kontrollierten Uterusmanipulation umfassend
- eine Führungsstange (1) mit einem proximalen zylindrischen stabförmigen Teil, ein zylindrisches distales Ende (4) und einen konischen Gewindeabschnitt (5) zwischen dem proximalen zylindrischen stabförmigen Teil (6) und dem zylindrischen distalen Ende (4),
- eine zervikale Schale (2), die koaxial entlang der Führungsstange bewegbar ist,
- eine zylindrische Sicherungsmutter (9),
wobei der mit einem Gewinde versehene konische Abschnitt der Führungsstange (1) so konfiguriert ist, dass er an dem Uterus durch den Zervikal Kanal befestigt ist,
wobei die zervikale Schale (2) einen Zylinder mit einem geschraubten proximalen Ende aufweist, das so konfiguriert ist, dass er an der Führungsstange an einem Befestigungspunkt befestigt werden kann, der individuell gewählt werden kann,
wobei die zervikale Schale weiterhin so konfiguriert ist, dass sie koaxial über den mit Gewinde versehenen konischen Abschnitt hoch bis zu den Scheidengewölben eingeführt wird,
dass das medizinische Instrument ferner umfasst eine birnenförmige Nuss (3), dass die zervikale Schale (2) ferner äußere gerillte Ränder (10) aufweist, um vaginale Fornices abzugrenzen und eine Pneumookklusion bereitzustellen,
wird **dadurch gekennzeichnet, dass** die zylindrische Sicherungsmutter (9) so konfiguriert ist, dass sie mit dem genannten geschraubten proximalen Ende des Zylinders mit der Zervikale Schale verschraubt werden kann, um den Zylinder an der Führungsstange zu befestigen,
dass die zylindrische Sicherungsmutter (9) ferner an ihrer Oberfläche eine äußere Schraube zur Befestigung der birnenförmigen Mutter (3) aufweist,
dass die birnenförmige Mutter (3) zur Okklusion der unteren Vagina so konfiguriert ist, dass sie koaxial zur äußeren Oberfläche der zylindrischen Sicherungsmutter fixiert ist.

2. Medizinisches Instrument zur kontrollierten Uterusmanipulation nach Anspruch 1,
**dadurch gekennzeichnet, dass** der Zylinder mit einer zervikalen Schale (2), die zylindrische Sicherungsmutter (9) und die birnenförmige Mutter (3) aus PTFE bestehe.

3. Medizinisches Instrument zur kontrollierten Uterusmanipulation nach Anspruch 1 und 2,
**dadurch gekennzeichnet, dass** die Führungsstange (1) entweder aus Edelstahl oder PTFE hergestellt ist.

## Revendications

1. Un instrument médical pour la manipulation contrôlée de l'utérus comprenant
- une tige de guidage (1) comprenant une partie cylindrique proximale en forme de tige, une extrémité distale cylindrique (4) et une partie conique filetée (5) entre la partie cylindrique proximale en forme de tige (6), et l'extrémité distale cylindrique (4),
- une coupelle cervicale (2) mobile coaxial le long de la tige de guidage
- un contre-écrou cylindrique (9),
dans lequel la partie conique filetée de la tige de guidage (1) est configurée pour être fixée à l'utérus à travers le canal cervical,
dans lequel la coupelle cervicale (2) comprend un cylindre avec une partie proximale vissée une extrémité configurée pour être fixée à la tige de guidage à un point de fixation qui peut être choisi individuellement,
la coupelle cervicale étant en outre configurée pour être introduite coaxial sur la partie conique filetée en amont des fornices vaginaux,
**caractérisé en ce que** l'instrument médical comprend en outre un écrou en forme de poire (3), **en ce que** la coupelle cervicale (2) comprend en outre des marges rainurées externes (10) pour délimiter des fornices vaginaux et fournir une pneumo occlusion,
**en ce que** l'écrou de blocage cylindrique (9) est configuré pour être vissé sur la dite extrémité proximale vissée du cylindre de la coupelle cervicale pour fixer le cylindre à la tige de guidage,
**en ce que** l'écrou de blocage cylindrique (9) comprend en outre une vis extérieure sur sa surface, pour la fixation de l'écrou en forme de poire (3),
**en ce que** l'écrou (3) en forme de poire est configuré pour l'occlusion du vagin inférieur et est configuré pour être fixé coaxial à la surface externe de l'écrou de blocage cylindrique.

2. Instrument médical pour la manipulation contrôlée de l'utérus selon la revendication 1,
**caractérisé en ce que** le cylindre avec une coupelle cervicale (2), l'écrou de blocage cylindrique (9) et l'écrou en forme de poire (3) sont en PTFE.

3. Instrument médical pour la manipulation contrôlée de l'utérus selon les revendications 1 et 2,
**caractérisé en ce que** la tige de guidage (1) est faite d'acier médical inoxydable ou de PTFE.
